# EUROPEAN PATENT APPLICATION

(11) **EP 1 958 957 A1**
(43) Date of publication of application: **20.08.2008**
(21) Application number: 07003331.1
(22) Date of filing: 16.02.2007
(51) Int. Cl.: C07K 14/00, A61K 38/16

(54) **Polypeptide comprising a knottin protein moiety**

(71) Applicant: NascaCell Technologies AG, 81377 München (DE)
(72) Inventor: Blind, Michael, 81245 München (DE); Kolmar, Harald, 64367 Mühltal (DE)
(74) Representative: Bohmann, Armin K.

(57) **Abstract**

The present invention is related to a polypeptide comprising a scaffold moiety and a helix moiety, whereby
the helix moiety is inserted into the scaffold moiety,
the scaffold moiety comprises a knottin protein or at least one fragment thereof,
and the amino acid sequence of the polypeptide differs from the amino acid sequence of the knottin protein or at least one fragment thereof.

## Description

The present invention is related to a polypeptide comprising a scaffold moiety and a helix moiety or a biologically active peptide moiety, a pharmaceutical composition comprising the same and use thereof.

Osteoporosis is a disease prevalent in postmenopausal women, but also affecting premenopausal women and men, associated with a reduction of the bone mineral density and disruption of the bone microarchitecture. It is a disorder of the skeleton that weakens bones leading to an increase in the risk of bone fracture, in particular in the spinal column, hip and wrist. The disease often develops silent und goes unnoticed over decades until a fracture occurs. Bones weakened by osteoporosis can fracture as a result of a minor fall or injury only that would not normally occur in non-osteoporotic people, leading to decreased mobility, pain and deformation.

A number of therapies are known that can be used to treat and/or prevent osteoporosis (see Hodsman, A. B., Bauer, D. C., Dempster, D. W., Dian, L., Hanley, D. A., Harris, S. T., Kendler, D. L., McClung, M. R., Miller, P. D., Olszynski, W. P., Orwoll, E., and Yuen, C. K. (2005), Endocrine Rev. 26, 688-703, for a review), among them bisphosphonates (Storm, T., Thamsborg, G., Steiniche, T., Genant, H. K., and Sorensen, O. H. (1990), N. Engl. J. Med. 322, 1265-1271; Watts, N. B., Harris, S. T., Genant, H. K., Wasnich, R. D., Miller, P. D., Jackson, R. D., Licata, A. A., Ross, P., Woodson, G. C., and Yanover, M. J. (1990), N. Engl. J. Med. 323, 73-39; Black, D. M., Cummings, S. R., Karpf, D. B., Cauley, J. A., Thompson, D. E., Nevitt, M. C., Bauer, D. C., Genant, H. K., Haskell, W. L., Marcus, R., Ott, S. M., Tomer, J. C., Quandt, S. A., Reiss, T. F., and Ensrud, K. E. (1996), Lancet 348, 1535-1541; Cummings, S. R., Black, D. M., Thompson, D. E., Applegate, W. B., Barrett-Connor, E., Musliner, T. A., Palermo, L., Prineas, R., Rubin, S. M., Scott, J. C., Bogt, T., Wallace, R., Yates, A. J., and LaCrois, A. Z. (1998), JAMA 280, 2077-2082), estrogen (Rossouw, J. E., Anderson, G. L., Prentice, R. L., LaCroiz, A. Z., Kooperberg, C., Stefanick, M. L., Jackson, R. D., Beresford, S. A., Howard, B. V., Johnson, K. C., Kotchen, J. M., and Ockene, J. (2002): Writing Group for the Womens's Health Initiative Investigators 2002, JAMA 288, 321-333), raloxifene (Ettinger, B., Black, D. M., Mitlak, B. H., Knickerbocker, R. K., Nickelsen, T., Genant, H. K., Christiansen, C., Delmas, P. D., Zanchetta, J. R. Stakkestad, J., Gluer, C. C., Krueger, K., Cohen, F. J., Eckert, S., Ensrud, K. E., Avioli, L. V., Lips, P., and Cummings, S. R. (1999), JAMA 282, 637-645), nasal calcitonin (Chesnut, C. H., Silverman, S., Andriano, K., Genant, H., Gimona, A., Harris, S., Kiel, D., LeBoff, M., Maricic, M., Miller, P., Moniz, C., Peacock, M., Richardson, P., Watts, N., and Baylink, D. (2000), Am. J. Med. 109, 267-276) and Parathyroid hormone (PTH) (Hodsman, A. B., Hanley, D. A., Ettinger, M. P., Bolognese, M. A., Fox, J., Metcalfe A. J., and Lindsay, R. (2003), J. Clin. Endocrinol. Metab. 88, 5212-5220).

PTH is a peptide comprising 84 amino acid residues and is responsible for the regulation of extracellular calcium homeostasis in blood and kidney (Chorev, M., Alexander, J., and Rosenblatt, M. (2001), In: The Parathyroids - Basic and Clinical Concepts (Bilezikian, J., Levine, M., and Marcus, R., eds), pp. 53-91. Raven Press, New York; M. Chorev, M., and Rosenblatt, M. (2002), In: Principles of Bone Biology (Bilezikian, J., Raisz, L., and Rodan, G.A., eds), pp. 423-461. Academic Press, San Diego, USA.). PTH acts on the PTH/PTHrP receptor (P1R), a class II G-protein-coupled receptor, which stimulates the adenylyl cyclase/cAMP and phospholipase C/inositol phosphate signaling pathways. Peptide deletion studies have shown that the *N-*terminal residues of PTH play a crucial role in P1R activation (Tregear, G. W., Van Rietschoten, J., Greene, E., Keutmann, H. T., Niall, H. D., Reit, B., Parsons, J. A., and Potts, J. T. (1973), Endocrinology 93, 1349; Takasu, H., Gardella, T. J., Luck, M. D., Potts, J. T., and Bringhurst, F. R. (1999), Biochemistry 38, 13453) and crosslinking. Receptor mutagenesis studies have revealed that the *N*-terminal residues of PTH interact with the portion of the P1R that contains the extracellular loops and extracellular ends of the transmembrane helices (Bergwitz, C., Gardella, T. J., Flannery, M. R., Potts, J. T., H. Kronenberg, M., Goldring, S. R., and Jüppner, H. (1996), J. Biol. Chem. 271, 26469; Hoare, S. R. J., Gardella, T. J., and Usdin, T. B. (2001), J. Biol. Chem. 276, 7741; V. Behar, A. Bisello, B. Bitan, M. Rosenblatt, M. Chorev (2000), J. Biol. Chem. 275, 9; M. D. Luck, P. H. Carter, T. J. Gardella (1999), Mol. Endocrinol. 13,670.).

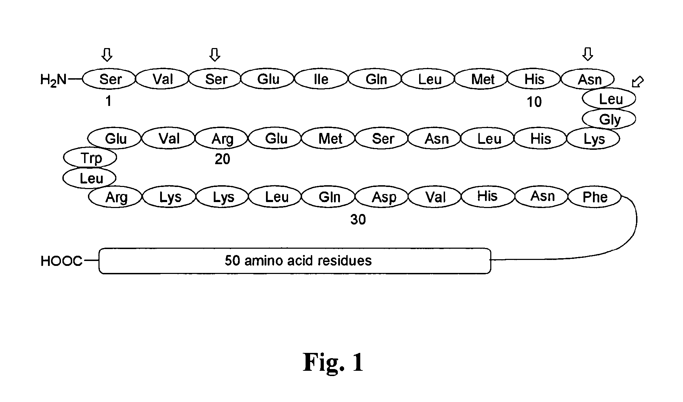

The *N*-terminal 1-34 sequence of PTH, as depicted in Fig. 1, which is also referred to as PTH (1-34), retains the full calciotropic activity of the intact PTH hormone. Clinical studies have demonstrated that PTH (1-34) is a powerful bone anabolic agent able to restore bone mineral density in postmenopausal women and to reduce fracture risk. Recently, recombinant human PTH (1-34), designated as *FORTEO^{®} (Teriparatide; Eli Lilly* and Company, Indianapolis, IN, USA), was approved by the FDA for treating osteoporosis in postmenopausal women who are at high risk for fractures.

The finding that PTH or N-terminal fragments thereof are agents suitable for the therapy of osteoporosis is somewhat surprising, as hyperparathyroidism, the pathological condition associated with the presence of an excess of PTH, for example as a result of a tumour, has been linked with bone loss, not bone gain. However, strictly, PTH does not cure osteoporosis, but greatly restores bone mass, increasing bone strength and dramatically reducing fracture incidence (Reeve, J., Meunier, P. J., Parsons, J. A., BErnat, M., Bijvoet, O. L., Courpron, P., Edouard, C., Klenerman, L., Neer, R. M., Renier, J. C., Slovik, D., Vismans, F. J., and Potts, J. T. (1980), British Medical Journal 280, 1340-1344; Tam, C. S., Heersche, J. M., Murray, T. M., and Parsons, J. A. (1982), Endocrinology 110, 506-512; Lindsay, R., Nieves, J., Formica, C., Henneman, E., Woelfert, L., Shen, V., Dempster, D., and Cosman, F. (1997), Lancet 350, 550-555; Lane, N. E., Sanchez, S., Modin, G. W., Genant, H. K., Pierini, E., and Arnaud, C. D. (1998), Journal of Clinical Investigation 102, 1627-1633; Dempster, D. W., Cosman, F., Kurland, E. D., Zhou, H., Nieves, J., Woelfert, L., Shane, E., Plavetic, K., Muller, R., Bilezikian, J., and Lindsay, R. (2001), Journal of Bone and Mineral Research 16, 1846-1853; Neer, R. M., Arnaud, C. D., Zanchetta, J. R., Prince, R., Gaich, G. A., Reginster, J. Y., Hodsman, A. B., Eriksen, E. F., Ish-Shalom, S., Genant, H. K., Wand, O., and Mitlak, B. H. (2001) Effect of parathyroid hormone (1-34) on fractures and bone mineral density in postmenopausal women with osteoporosis. New England Journal of Medicine 344, 1434-1441). PTH(1-84) has received marketing approval within the EU and is known as Preotact^{®}. The mechanism of action relates to the PTH receptor, which is present only on the osteoblast; its activation by the hormone may prolong osteoblast life and increase its activity leading to bone formation. The signalling by several intermediate messengers from osteoblast to osteoclast to stimulate the latter and resorb bone occurs less rapidly than the initial direct stimulation of the osteoblast. Hence, elevations of the PTH levels that are transient may stimulate osteoblast anabolic activity while not yet triggering the coupled catabolic response through osteoclasts (Potts, J. T. (2005), Journal of Endicronology 187, 311-325). Timing appears to be critical in the administration of PTH. An important study (Dobing, H., and Turner, R. T. (1997), Endocrinology 138, 4607-4612) in test animals with controlled rates of administration of hormone showed that less than two hours of exposure to PTH elicited the anabolic response and longer than two hours the catabolic response. Therefore, while the exact cellular mechanisms by which the favourable anabolic response occurs as a result of a short exposure to increased PTH levels are unknown, it is well established that intermittent doses of PTH, for example subcutaneous administration of 20 µg daily of recombinant PTH (1-34), in contrast to continuous doses of the peptide, are beneficial (Compston, J. E (2006), Bone 2006 Oct 11, electronic publication ahead of print).

Conformational studies by NMR and structural calculations on PTH (1-34) analogues in polar and nonpolar solvents (Marx, U. C., Austermann, S., Bayer, P., Adermann, K., Eschart, A., Stich, H., Walter, S., Schmid, F. X. , Janicke, R., Forssmann, W. G., and Rosch, P. (1995) J. Biol. Chem. 270, 15194; Marx, U. C., Adermann, K., Bayer, P., Meyer, M., Forssmann, W. G., Rosch, P. (1998), J. Biol. Chem. 273, 4308; M. Pellegrini, A. Bisello, M. Rosenblatt, M. Chorev, D. F. Mierke (1998), Biochemistry 37, 12737) suggest that the *N*-terminal portion of PTH, known to be responsible for the activation of the transmembrane hepta-helical G-protein-coupled receptor, contains a short, 10 amino acid residues, helical segment. Moreover, it has been reported that the shorter analog PTH (1-14) is able to activate cAMP formation at high micromolar concentrations in cells (Shimizu, M., Potts, J. T., Gardella, T. J. (2000), J. Biol. Chem. 275, 21836). The potency of PTH (1-14) can be markedly increased through the introduction of specific amino acid substitutions. The homo-arginine (Har) containing analogue is 40-fold more potent than the native PTH (1-14)-NH₂. Recently, the introduction of the Cα-tetrasubstituted, sterically hindered α-amino isobutyric acid (Aib) was shown to strongly increase the potency of PTH-(1-14)-NH₂ and even of the shorter sequence PTH (1-11)-NH₂. Shimizu, N., Guo, J., Gardella, T. J. (2001), J. Biol. Chem. 276, 49003). Aib and related chiral and achiral analogs and homologs are widely known to facilitate stable helix formation in oligopeptides, as exemplified by a large number of crystal structure determinations by X-ray analysis Toniolo, C., and Benedetti, E. (1991), Trends Biochem. Sci. 1991, 16, 350; Karle, L., and Balaram, P. (1990), Biochemistry 29, 6747; Kaul, R., and Balaram, P. (1999): Stereochemical control of peptide folding. Bioorg. Med. Chem. 7,105).

In line with the previous finding that swapping some of the residues in the *N*-terminal sequence of the natural PTH for peptide helicity-increasing residues (Gln, Aib, Har) results in an enhanced potency of the binding to PTH-1 receptor (P1R), it has been suggested that the α-helical structural motif may foster the interactions with the PTH/P1R receptor (Shimizu, N., Petroni, B. D., Khatri, A., and Gardella, T.J. (2003), Biochemistry 42, 2282; Tsomaia, N., Pellegrini, M., Hyde, K., Gardella, T. J., Mierke, D. F. (2004), Biochemistry 43, 690).

However, consistent with the idea that short and largely unstructured peptides are readily degraded by cellular proteases, the half lives of PTH and minimized PTH peptides in human plasma are extremely short. Consequently, FORTEO^{®} has to be applied daily through subcutaneous injection, a rather inconvenient way of application, which requires a significant degree of effort both on part of the patient and the clinician.

Therefore, a problem underlying the present invention is to provide a means which allows the administration of a peptide which is preferably a biologically active peptide, to a biological system such as an organism.

A further problem underlying the present invention is to provide a means which allows to protect a peptide which is preferably a biologically active peptide and/or an unstructured peptide, from being rendered biologically inactive, whereby such inactivation may occur through degradation of the peptide or removal thereof from a biological system such as an organism to which said peptide has been administered.

These and other problems are solved by the present invention and the independent claims attached hereto. Preferred embodiments may be taken from the dependent claims.

The problem underlying the present invention is solved in a first aspect by a polypeptide comprising a scaffold moiety and a helix moiety, whereby
the helix moiety is inserted into the scaffold moiety,
the scaffold moiety comprises a knottin protein or at least one fragment thereof,
and the amino acid sequence of the polypeptide differs from the amino acid sequence of the knottin protein or at least one fragment thereof.

The problem underlying the present invention is solved in a second aspect by a polypeptide comprising a scaffold moiety and a biologically active peptide moiety, whereby
the biologically active peptide moiety is inserted into the scaffold,
the scaffold moiety comprises a knottin protein or at least one fragment thereof,
and the amino acid sequence of the polypeptide differs from the amino acid sequence of the knottin protein or at least one fragment thereof.

In an embodiment of the first and the second aspect of the present invention the polypeptide and/or the scaffold moiety is cyclic.

In a further embodiment of the first and the second aspect of the present invention the polypeptide and/or the scaffold moiety is linear.

In an embodiment of the first and the second aspect of the present invention the knottin protein is selected from the group comprising EETI-II M7I, oMcoTI-II, McoEeTI, AGRP' and Obtustatin.

In a further embodiment of the first and the second aspect of the present invention the helix moiety comprises an amino acid sequence, whereby such amino acid sequence is one of a biologically active peptide, whereby preferably such biologically active peptide is selected from the group comprising peptide hormones, cytokines, integrins, integrin ligands, protease inhibitors, GPCR ligands, ion chanel ligands, DNA or RNA ligands, viral proteins, bacterial proteins or a fragment and/or derivative thereof.

In an embodiment of the first and the second aspect of the present invention the biologically active peptide moiety comprises an amino acid sequence whereby such amino acid sequence is one of a peptide selected from the group comprising peptide hormones, cytokines, integrins, protease inhibitors, viral proteins, bacterial proteins, or a fragment thereof and/or derivative thereof.

In an embodiment of the first and the second aspect of the present invention the amino acid sequence of the scaffold moiety comprises at least two, preferably at least four and more preferably six Cys residues.

In an embodiment of the first and the second aspect of the present invention the amino acid sequence of the scaffold moiety comprises six or eight cysteines.

In an embodiment of the first and the second aspect of the present invention the helix moiety or the biologically active peptide moiety is inserted into the scaffold moiety between two Cys redidues of the knottin protein of the scaffold moiety.

In an embodiment of the first and the second aspect of the present invention the helix moiety or the biologically active peptide moiety is inserted into the scaffold moiety, counting from the N-terminus to the C-terminus, between the first and the second, the fourth and the fifth or the fifth and the sixth Cys residue of the scaffold moiety.

In an embodiment of the first and the second aspect of the present invention the scaffold moiety is derived from the knottin protein by having deleted at least one, preferably more and most preferably all of the amino acid residues between the Cys residues of the knottin protein or a fragment thereof between which the helix moiety or the biologically active peptide moiety is inserted.

In a preferred embodiment all of the amino acid residues between the Cys residues of the knottin protein or the fragment thereof are deleted.

In an embodiment of the first and the second aspect of the present invention the polypeptide comprises or consists of a structure which is as follows:
the knottin protein is EETI-II M7I and the amino acid sequence between the first and the second cysteine of the knottin protein is partially or in its entirety replaced by the amino acid sequence of the helix moiety or the biologically active peptide moiety, or
the knottin protein is oMcoTI-II and the amino acid sequence between the first and the second cysteine of the knottin protein is partially or in its entirety replaced by the amino acid sequence of the helix moiety or the biologically active peptide moiety, or
the knottin protein is McoEeTI and the amino acid sequence between the first and the second cysteine of the knottin protein is partially or in its entirety replaced by the amino acid sequence of the helix moiety or the biologically active peptide moiety, or
the knottin protein is AGRP' and the amino acid sequence between the fifth and the sixth cysteine of the knottin protein is partially or in its entirety replaced by the amino acid sequence of the helix moiety or the biologically active peptide moiety, or
the knottin protein is Obtustatin and the amino acid sequence between the fourth and the fifth cysteine of the knottin protein is partially or in its entirety replaced by the amino acid sequence of the helix moiety or the biologically active peptide moiety.

In an embodiment of the first and the second aspect of the present invention the helix moiety or the biologically active peptide moiety is fused to the N-terminus or the C-terminus of the scaffold moiety.

In an embodiment of the first and the second aspect of the present invention the biologically active peptide is selected from the group comprising PTH, PTH derivatives and PTH analogues, preferably selected from the group comprising a peptide having an amino acid sequence according to SEQ. ID No. 1, a peptide having an amino acid sequence according to SEQ. ID No. 2, a peptide having an amino acid sequence according to SEQ. ID. No. 3 and a peptide having an amino acid sequence according to SEQ. ID. No. 12, and fragments and/or derivatives thereof.

In an embodiment of the first and the second aspect of the present invention the scaffold moiety is oMcoTI-II according to SEQ ID No 4 and the helix moiety or the biologically active peptide moiety is a peptide having an amino acid sequence according to SEQ ID No. 2 or an amino acid according to SEQ. ID. No. 12, with the helix moiety of the biologically active peptide moiety being inserted between the first cysteine and the second cysteine of the amino acid sequence according to SEQ ID No 4, replacing the amino acid residues occurring between said two cysteines of the amino acid sequence of the knottin protein.

In an embodiment of the first and the second aspect of the present invention the scaffold moiety is oMcoTI-II according to SEQ ID No 4 and the helix moiety or the biologically active peptide moiety is a peptide having an amino acid sequence according to SEQ ID No. 3, with the helix moiety of the biologically active peptide moiety being inserted between the first cysteine and the second cysteine of the amino acid sequence according to SEQ ID No 4, replacing the amino acid residues naturally occurring between said two cysteines of the amino acid sequence of the knottin protein.

In an embodiment of the first and the second aspect of the present invention the length of the helix moiety and/or the biologically active peptide moiety is from about 4 to 30 amino acids, preferably from about 4 to 25 amino acids, more preferably from about 4 to 20 amino acids, and even more preferably from about 4 to 15 amino acids.

In an embodiment of the first and the second aspect of the present invention the polypeptide is a recombinant protein.

In an embodiment of the first and the second aspect of the present invention, the polypeptide comprises or consists of an amino acid sequence according to SEQ. ID. No. 6, SEQ. ID. No. 7 or SEQ. ID. No. 13.

In a further embodiment of the first and the second aspect of the present invention the polypeptide is a chemically synthesised protein or a synthetic protein.

In an embodiment of the first and the second aspect of the present invention the scaffold moiety comprises one knottin protein or at least one fragment thereof.

In a further embodiment of the first and the second aspect of the present invention the scaffold protein comprises a multimer of a knottin protein or of at least a fragment thereof, preferably a dimer.

In an embodiment of the first and the second aspect of the present invention the knottin protein or at least one fragment thereof is a knottin protein or at least one fragment thereof as defined in any embodiment of the first and the second aspect of the present invention.

The problem underlying the present invention is solved in a third aspect by a pharmaceutical composition comprising a polypeptide according to the first and/or the second aspect of the present invention, and a pharmaceutically acceptable carrier.

In an embodiment of the third aspect of the present invention the pharmaceutical composition is for oral administration.

The problem underlying the present invention is solved in a fourth aspect by the use of the polypeptide according to the first and/or the second aspect of the present invention for the manufacture of a medicament for the treatment or the prevention of a disease.

The problem underlying the present invention is solved in a fifth aspect by the use of the polypeptide according to the first and/or the second aspect of the present invention for the manufacture of a diagnostic agent for the diagnosis of a disease.

In an embodiment of the fourth and the fifth aspect of the present invention the disease is bone-related disorder.

In an embodiment of the fourth and the fifth aspect of the present invention the bone-related disorder is a bone disorder characterized by low bone mineral density (BMD) and/or bone fragility.

In an embodiment of the fourth and the fifth aspect of the present invention the bone disease is selected from the group comprising primary and secondary osteoporosis, osteopenia, osteomalacia, osteogenesis imperfecta (OI), avascular necrosis (osteonecrosis), fractures and implant healing, and bone loss due to other disorders.

In an embodiment of the fourth and the fifth aspect of the present invention the other disorder resulting in bone loss is selected from the group comprising HIV infection, cancers and arthritis.

In a further embodiment of the fourth and the fifth aspect of the present invention the implant healing is the implant healing of dental implants or hip implants.

In an embodiment of the fourth and the fifth aspect of the present invention the bone-related disorder is selected from the group comprising osteoarthritis, arthritis, and the formation and presence of osteolytic lesions.

The present inventors have surprisingly found that a polypeptide comprising a scaffold moiety and a helix moiety and more preferably an alpha helix moiety or a biologically active peptide moiety, whereby the helix moiety or the biologically active peptide moiety is inserted into the scaffold moiety, the scaffold moiety comprises a knottin protein or at least one fragment thereof, and the amino acid sequence of the polypeptide differs from the amino acid sequence of the knottin protein or at least one fragment thereof, is suitable to stably present the helix moiety or the biologically active peptide moiety.

As used herein, a helix moiety is preferably a moiety comprising a helix or it consists of a helix. In a preferred embodiment, the helix is an alpha helix.

As used herein, a biologically active peptide moiety is a moiety comprising a biologically active peptide or consists of a biologically active peptide.

In a preferred embodiment, the term "helix", as used herein, or related terms such as "helical", refer to an alpha helix. Alpha helices are a common secondary structural motif of proteins found in globular and fibrous proteins. The average length of an alpha helix in a globular protein is 11 amino acids but can extend to over 50 amino acids in some cases. The helix contains 3.6 amino acids per turn and the hydrogen bonds are arranged such that the peptide C=O bond of the nth moiety points to the N-H goup of the (nth+4 moiety). (Voet and Voet, Biochemistry,1990, Wiley and Sons Inc., p. 149-150).

Even more surprisingly the present inventors have realised that such helix moiety, if it is showing some biological effect as such, and the biologically active peptide moiety having some biological effect as such, retain said effect when being part of the polypeptide of the present invention. Likewise, the present inventors have surprisingly found that the functionality of the knottin protein is retained upon insertion of the helix moiety. In a preferred embodiment, the "functionality of the knottin protein", as used herein, refers to a state of structural and functional properties essentially unaltered in comparison to the respective wild type protein.

Without wishing to be bound by any theory, the present inventors have discovered that the use of a knottin protein or a fragment thereof is suitable to act as a scaffold for such helix moiety, i. e. preferably a peptide having some helix as a secondary structure, and such biologically active peptide, respectively, while not interfering with the effect or activity thereof. Furthermore, the present inventors have discovered that retaining the effect or activity in such molecular environment goes typically along with a stabilisation of the moiety and peptide, respectively. Such stabilisation is preferably indicated by an increased lifetime of the helix and peptide, respectively, compared to the stabilisation of the helix and the peptide, respectively, if not forming part of the polypeptide according to the present invention.

Specifically with regard to PTH and its analogs the present inventors have surprisingly found that the biologically active PTH fragments such as PTH (1-11), herein also referred to as PTH-2 and having an amino acid sequence of Aib-Val-Aib-Glu-Ile-Gln-Leu-Met-His-Gln-Har (SEQ. ID. No. 3), as well as PTH (1-14), herein also referred to as PTH-1 and having an amino acid sequence of Aib-Val-Aib-Glu-Ile-Gln-Leu-Met-His-Gln-Har-Ala-Lys-Trp (SEQ. ID. No. 2), and PTH-1*, having an amino acid sequence of Aib-Val-Aib-Glu-Ile-Gln-Leu-Met-His-Gln-Har-Ala-Lys-Tyr (SEQ. ID. No. 12), can be stably inserted into knottin scaffold moieties while retaining their biological activity. In a preferred embodiment of the invention, the scaffold moiety is provided by the rigid molecular scaffold of a inhibitor cystine knot (ICK) polypeptides (knottins).

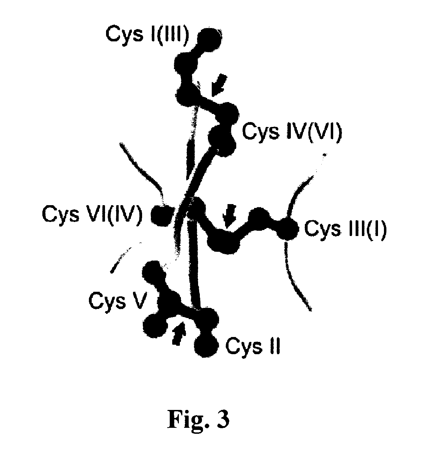

Knottins which are also referred to as Cystine knot miniproteins in the art and as knottin proteins or microproteins herein, represent a class of proteins that are typically composed of less than 40 amino acids and are typically characterized by a defined structure consisting of an arrangement of three disulfide bonds forming a cystine knot, a small triple-stranded β-sheet and a short 3₁₀ helix as depicted in Fig. 3 (Craik, D. J., Daly, N. L., and Waine, C. (2001), Toxicon 39, 43-60). In a preferred embodiment of the present invention, any "cysteine" addressed in any context of this document, more preferably in the context of the insertion of a helix moiety or biologically active peptide moiety between cysteines, is one of the natural disulfide bond-forming cysteines of the respective knottin. Herein, cysteines are also referred to as "Cys" in accordance with the widely accepted textbook abbreviation. Knottins display a plethora of different biological activities in nature ranging from protease inhibition in plants belonging to the squash family, triggering of signal transduction events in humans to ion channel blockage by members of conotoxins which are extremely potent toxins produced by marine cone snails.

The cystine knot appears to be a highly efficient motif for structure stabilization. The autonomous folding unit of knottins is the elementary two-disulfide motif, the so-called Cystine stabilized beta-sheet. The defined structure of knottins renders them extremely stable against pH, temperature and proteolytic attack, an effect that is particularly pronounced in some variants that possess a cyclic backbone through linkage of their N- and C-termini *via* an additional peptide bond (Colgrave, M. L., and Craik, D. J. (2004), Biochemistry 43, 5965-5975).

Exemplary knottin proteins are EETI-II M7I (also referred to as "-ET") which is a variant of the trypsin inhibitor EETI-II from the seeds of the cucumber plant *Ecbalium elaterium* (Heitz, A., Chiche, L., Le-Nguyen, D. & Castro, B., Biochemistry, 1989. 28(6): p. 2392-8), oMcoTI-II (also referred to as "-MC") which is a linear variant of the naturally cyclic trypsin inhibitor MCoTI-II from the seeds of *Momordica cochinchinensis* (Avrutina, O., Schmoldt, H.U., Kolmar, H. & Diederichsen, U., Eur J Org Chem, 2004(23): p. 4931- 4935), McoEeTI (also referred to as "-MG") which is a hybrid miniprotein consisting of the aminoterminal part of McoTI and the carboxyterminal part of EETI (Schmoldt, H.U., Wentzel, A., Becker, S. & Kolmar, H., Protein Expr Purif, 2005. 39(1): p. 82-89), AGRP' (also referred to as "-AG") which is a rationally minimized miniprotein derived from the human agouti-related protein which is an endogenous antagonist of the hypothalamic melanocortin receptor with orexigen effects (Jackson, P.J., McNulty, J.C Yang, Y.K., Thompson, D.A., Chai, B., Gantz, I., Barsh, G.S. & Millhauser, G.L., Biochemistry, 2002. 41(24): p. 7565-72) and Obtustatin (also referred to as "-OB") which is a disintegrin initially isolated from the venom of the Vipera lebetina obtusa viper (Paz-Moreno-Murciano, M., Monieon, D., Marcinkiewicz, C., Calvete, J.J. & Celda, B., J Mol Biol, 2003 May 23; 329(1):135-45). The protein does not contain the classical RGD sequence characteristic of other integrins.

In a preferred embodiment, the term "scaffold moiety", as used herein, refers to a protein or polypeptide that adopts a stable tertiary and/or quaternary structure, either by itself or in combination with the helix moiety or the biologically active peptide moiety. The person skilled in the art is aware of ways to quantify the stability of such constructs, for example through the use of biophysical methods, such as melting experiments monitored by techniques such as Circular Dichroism spectroscopy, calorimetry or Nuclear Magnetic Resonance spectroscopy, or through functional assays detecting, for example, the resistance of the construct in question to proteolytic digestion. In another particularly preferred embodiment, the scaffold moiety comprises at least two, preferably at least four and more preferably six or even more cystein residues. In another particularly preferred embodiment, the scaffold moiety comprises six or eight cysteines. Typically said Cys residues increase the stability of the polypeptide through formation of disulfide bonds. Such disulfide bonds may form naturally occurring cysteines or cysteines introduced through genetic engineering or in any combination thereof. The person skilled in the art is able to introduce such additional disulfide bonds based on the structural information available as particularly mentioned in other contexts in this specification. Moreover, the person skilled in the art is able to modify the primary sequence, e.g. the N-terminus, in a way that decreases the degree of degradation by the prokaryotic or eukaryotic cellular degradation machineries. The person skilled in the art is also aware of methods for the synthesis, expression and purification of such polypeptides. Methods for expression can include a modification of the primary structure, for example the use of affinity tags such as His tags, GST tags or MBP tags, most often in combination with a protease cleavage site for subsequent removal of the tag, or epitope tags for the isolation or detection of the polypeptide or complexes comprising the polypeptide using suitable antibodies.

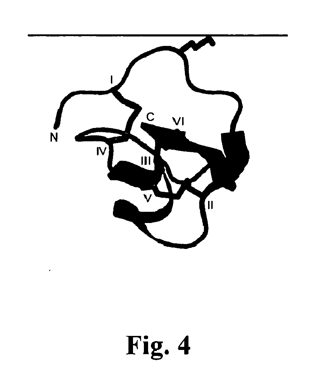

In an embodiment of the present invention, the scaffold moiety comprises a knotting protein. In a preferred embodiment of the present invention, the knottin is selected from the group comprising EETI-II M7I (-ET), oMcoTI-II (-MC), McoEeTI (-MG), AGRP' (-AG) and Obtustatin (-OB). oMcoTI-II was derived from naturally cyclic squash inhibitor MCoTI-II isolated from the seeds of *Momordica cochinchinensis* by virtue of its ability to inhibit trypsin. The only difference between these two microproteins is that oMcoTI-II does not have the part of the cyclization loop *(i.e.* amino acid residues 28-32). This very microprotein was used as a starting point. As will be described in more detail herein, the helical motif of PTH (1-11) and PTH (1-14) was introduced between cysteines I and II of the oMcoTI-II protein (Fig. 4) yielding PTH Microbodies^{™} NC-MC-PTH-1 and -2.

In one embodiment, the polypeptide of the present invention and/or the scaffold is cyclic, which can preferably be achieved through the connection of the N-terminus and the C-terminus, for example *via* an additional peptide bond. However, in principle, a suitable amino acid residue side chain can also be chemically modified to allow for formation of a cyclic polypeptide and/or scaffold moiety. In an alternative embodiment, the polypeptide and/or scaffold moiety is linear, which includes all kinds of branched molecules that can be produced by chemically modifying amino acid residue side chains. It is also within the present invention that the polypeptide of the present invention is linear or non-cyclic, whereas the scaffold moiety is cyclic, and vice versa. Additionally, it is also within the present invention that the polypeptide or the scaffold moiety is linear and the helix moiety or biologically active peptide moiety is cyclic. It will be acknowledged that the scaffold moiety as used herein is a knottin protein, a fragment thereof or a derivative thereof. A fragment or derivative of a knottin protein is preferably a fragment or derivative of a knottin protein under the proviso that such fragment or derivative is still functionally active as a knottin protein, at least functionally active to the extent that the polypeptide of the present invention comprising such fragment or derivative has at least one of the characteristics of the polypeptide of the present invention comprising a full-length knottin protein or a scaffold derived from such full length knottin protein.

Knottin proteins are typically monomeric, and may be used as such in an embodiment of the polypeptide of the present invention. However, in an embodiment of the present invention, the scaffold protein comprises a multimer of or is formed of more than one monomer of such a knottin protein or at least a fragment thereof, preferably a dimer. In a preferred embodiment of the invention, a "multimer", as used herein, is a complex comprising more than one molecule of a species, also referred to herein as monomer. A "dimer", as preferably used herein, comprises a complex consisting of two molecules of the same species. Such a complex may be a covalent or non-covalent complex. In an embodiment a helix moiety or a biologically active peptide moiety is inserted into one or several of such monomers.

Preferably, the scaffold moiety is a protein or polypeptide derived from a knottin protein. The derivatisation is such that starting from the amino acid sequence of the knottin protein one, more than one or all of the amino acids are deleted which are present between two Cys residues of the knottin protein.

In a preferred embodiment, the term "helix moiety", as used herein, refers to a sequence of amino acids forming a helix or having an at least partially helical character. Such amino acids, individually or in any combination may be naturally occurring amino acid, proteinogenic amino acids or non-naturally occurring amino acids. Preferably such amino acid promotes the formation of a helix when being incorporated into an amino acid sequence naturally, or derivatives thereof. The person skilled in the art is aware that such a moiety is not necessarily 100% helical, but may also comprise unstructured parts or even other secondary structure elements. In another preferred embodiment, the helix moiety comprises natural or unnatural amino acids known as peptide helicity-increasing residues such as, but not limited to, Gln, Aib, Har. The person skilled in the art is able to introduce unnatural amino acids into peptide or polypeptides, for example through the chemical synthesis of such peptides or polypeptides using suitable amino acid precursors, by feeding unnatural amino acids or precursors thereof to organisms expressing the peptide or polypeptide in question or through the use of tRNA molecules chemically loaded with such unnatural amino acids or precursors thereof. An amino acid as preferably used herein is an chemical compound comprising both a carboxy and an amino group and that can preferably be incorparated into the primary sequence of a peptide or polypeptide.

The helix moiety is in principle not restricted in terms of size or function. In an embodiment the helix moiety even comprises a complete folded polypeptide or a fragment thereof as long as the biological activity or biological effect is retained upon insertion into the scaffold moiety and thus into the polypeptide of the present invention and the stability thereof is not compromised. In a particularly preferred embodiment, the biologically active peptide moiety or the helix moiety comprises an amino acid sequence, whereby such amino acid sequence is one of a biologically active peptide, whereby preferably such biologically active peptide is selected from the group comprising peptide hormones, cytokines, integrins, integrin ligands, protease inhibitors, GPCR ligands, ion channel ligands, DNA or RNA ligands, viral proteins, bacterial proteins or a fragment and/or derivative thereof.

In a preferred embodiment, the term "biologically active peptide moiety", as referred to herein, comprises a peptide moiety that is able to elicit some biological effect in a biological system. The biologically active peptide moiety is in principle not restricted in terms of size or function. In one embodiment, the helix moiety comprises a complete folded polypeptide or a fragment thereof as long as the biological activity or biological effect thereof is retained upon insertion into the scaffold and the stability of the scaffold is not compromised.

It will be acknowledged that the helix moiety preferably also has may have a biological activity or a biological effect similar to the biologically active peptide moiety. Such biological activity is preferably a biological effect. A biological effect as preferably used herein is any effect selected from the group comprising antigene effect, inhibiting a receptor or another biologically active molecule, immunostimulatory effect, receptor binding effect, triggering a signal cascade, and conveying biological information.

In a preferred embodiment, the helix and the helix moiety, respectively, comprises about 4 to about 30 amino acids. In a more preferred embodiment, the helix and the helix moiety, respectively, comprises 4 to about 25 amino acids, in an even more preferred embodiment the helix and the helix moiety, respectively, comprises 4 to about 20 amino acids, and in a most preferred embodiment, the helix and the helix moiety, respectively, comprises 4 to about 15 amino acids. The same considerations in terms of size are also applicable to the biologically active peptide and the biologically active peptide moiety, respectively.

Finally, it is within the present invention that the helix moiety comprises or represents a biologically active peptide, and that the biologically active peptide moiety comprises or consists of a helix.

In a particularly preferred embodiment, the biologically active peptide moiety or the helix moiety is selected from the group comprising peptide hormones, cytokines, integrins, integrin ligands, protease inhibitors, GPCR ligands, ion chanel ligands, DNA or RNA ligands, viral proteins, bacterial proteins or a fragment and/or derivative thereof. In an even more preferred embodiment, the biologically active peptide moiety is selected from the group comprising PTH, PTH derivatives and PTH analogues, preferably selected from the group comprising a peptide having an amino acid sequence according to SEQ. ID No. 1 a peptide having an amino acid sequence according to SEQ. ID No. 2 and a peptide having an amino acid sequence according to SEQ. ID No. 12 and a peptide having an amino acid sequence according to SEQ. ID. No. 3 and PTH(1-34) and fragments and/or derivatives thereof.

In a particularly preferred embodiment of the invention, the scaffold moiety is oMcoTI-II according to SEQ ID No 4 and the helix moiety or the biologically active peptide moiety is a peptide having an amino acid sequence according to SEQ ID No. 2 or SEQ ID No.12, with the moiety of the biologically active peptide moiety being inserted between Cys 1 and Cys 2 of the amino acid sequence according to SEQ ID No 4, replacing all the the amino acid residues occurring between said two cysteines of the amino acid sequence of the knottin protein. The resulting polypeptides have the following amino acid sequence.
(NH2)-G-V-C-**(Aib)-V-(Aib)-E-I-Q-L-M-H-Q-(Har)-A-K-W**-C-R-R-D-S-D-C-P-G-A-C-I-C-R-G-N-G-Y-C-G (COOH) (SEQ ID NO 6)
(NH2)-G-V-C-**(Aib)-V-(Aib)-E-I-Q-L-M-H-Q-(Har)-A-K-Y-**C-R-R-D-S-D-C-P-G-A-C-I-C-R-G-N-G-Y-C-G (COOH) (SEQ ID NO 13)

In another particularly preferred embodiment of the invention, the scaffold moiety is oMcoTI-II according to SEQ ID No 4 and the helix moiety or the biologically active peptide moiety is a peptide having an amino acid sequence according to SEQ ID No. 3, with the helix moiety of the biologically active peptide moiety being inserted between cysteine 1 and cysteine 2 of the amino acid sequence according to SEQ ID No 4, replacing all of the amino acid residues naturally occurring between said two cysteines of the amino acid sequence of the knottin protein. The resulting polypeptide has the following amino acid sequence.
(NH2)-G-V-C-**(Aib)-V-(Aib)-E-I-Q-L-M-H-Q-(Har)-**C-R-R-D-S-D-C-P-G-A-C-I-C-R-G-N-G-Y-C-G (COOH) (SEQ ID NO 7)

In accordance with the present invention, the helix moiety or the biologically active peptide moiety can be inserted into the scaffold moiety at various positions. A preferred position is between two cysteine residues of the scaffold moiety. In a more preferred embodiment, the helix moiety or the biologically active peptide moiety is inserted between the first and the second cysteine residue of the scaffold moiety. In connection with the numbering of the Cys residue it is to be noted that the counting of the Cys residues starts from the N-terminal of the polypeptide and scaffold moiety, respectively. Accordingly, the aminoterminal amino acid residue is the first, the amino acid residues linked to the C-terminus of the first residue is the second and so on. In another embodiment, all of the amino acids between the two cysteine residues prior to insertion of the helix moiety or the biologically active peptide moiety are deleted. In another embodiment of the present invention, the helix moiety or the biologically active peptide moiety is fused to the N-terminus or the C-terminus of the scaffold moiety.

In a preferred embodiment of the polypeptide of the present invention, the knottin protein is EETI-II M7I and the amino acid sequence between the first and the second cysteine of the knottin protein is completely or partially replaced by the amino acid sequence of the helix moiety or the biologically active peptide moiety. In another preferred embodiment of the polypeptide of the present invention, the knottin protein is oMcoTI-II and the amino acid sequence between the first and the second cysteine of the knottin protein is completely or partially replaced by the amino acid sequence of the helix moiety or the biologically active peptide moiety. In another preferred embodiment of the polypeptide of the present invention, the knottin protein is McoEeTI and the amino acid sequence between the first and the second cysteine of the knottin protein is completely or partially replaced by the amino acid sequence of the helix moiety or the biologically active peptide moiety. In another preferred embodiment of the polypeptide of the present invention, the knottin protein is AGRP' and the amino acid sequence between the fifth and the sixth cysteine of the knottin protein is replaced by the amino acid sequence of the helix moiety or the biologically active peptide moiety. In another preferred embodiment, the knottin protein is Obtustatin and the amino acid sequence between the fourth and the fifth cysteine of the knottin protein is completely or partially replaced by the amino acid sequence of the helix moiety or the biologically active peptide moiety.

In one embodiment of the invention, the polypeptide is a recombinant polypeptide , *i.e*. an expression vector comprising a nucleic acid encoding the polypeptide is used to transform a suitable expressing organism such as *E*. *coli,* yeast or mammalian cell lines, and the protein is purified from cultures of the expressing organism. In another embodiment, the polypeptide is a chemically synthesised protein or a synthetic protein such as one synthesised in vitro by solid phase synthesis methods. The respective expression vector is a further aspect of the present invention.

In a further aspect, the present invention relates to a pharmaceutical composition comprising the polypeptide of the invention and a pharmaceutically acceptable carrier.

Such a pharmaceutical composition comprises a therapeutically effective amount of the polypeptide of the present invention or a nucleic acid molecule coding therefore under the proviso that the polypeptide consists of proteinaceous amino acid and, optionally, a pharmaceutically acceptable carrier. The pharmaceutical composition may be administered with a physiologically acceptable carrier to a patient, as described herein. In a embodiment, the term "pharmaceutically acceptable" means approved by a regulatory agency or other generally recognized pharmacopoeia for use in animals, and more particularly in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides.

A preferred embodiment of the composition according to the invention is an oral composition, i. e. a composition which is intended for oral administration. Again without wishing to be bound by any theory, the present inventors assume that the high stability of the polypeptide according to the present invention makes them prone for oral administration and oral pharmaceutical formulations, respectively. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W.). Such compositions will contain a therapeutically effective amount of the polypeptide of the invention, preferably in purified form, together with a suitable amount of carrier so as to provide the form for proper administration to the patient. The formulation should suit the mode of administration. In a preferred embodiment of the invention, the pharmaceutical composition is for oral administration.

In a further aspect, the problem underlying the present invention is solved by using the polypeptide of the present invention for the manufacture of a medicament for the treatment or the prevention of a disease. In another aspect, the problem underlying the present invention is solved by using the polypeptide for the manufacture of a diagnostic agent for the diagnosis of a disease. In a preferred embodiment of both aspects, the disease is osteoporosis. In a preferred embodiment of the present invention, the polypeptide comprises PTH or a fragment and/or derivative thereof as the helix moiety and the biologically active peptide moiety, respectively, and is for intermittent administration.

Any diagnosis, similar to any therapeutic effect arising from the polypeptide of the invention is preferably mediated by the helix moiety and/or the biologically active peptide moiety. Said moiety interacts with an interaction partner which is preferably an interaction partner with which the moiety, as such, would also be reactive and interacting. This interaction triggers than a reaction which is responsible for the therapeutic effect or the detection of the interaction partner or any of the effects of the interaction partner.

The present invention is further illustrated by the following figures and examples from which further features, embodiments, aspects and advantages of the present invention may be taken, whereby
Fig. 1 is a representation of the human PTH with the teriparatide sequence (1-34) indicated, i.e. the sites commonly used for the modification in drug design are altered.

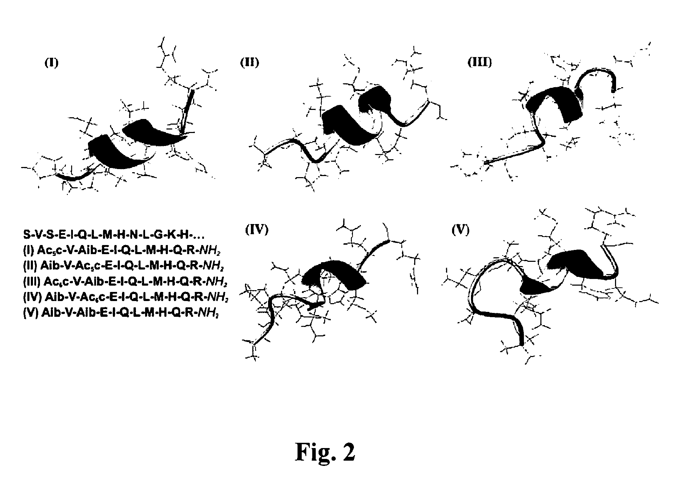

Fig. 2 shows sequences of the short α-helical peptide PTH (1-11) and its analogues (I-V) with their lowest energy structures are given; abbreviations: Aib, α-amino iso-butyric acid, Ac₅c, 1-aminocyclopentane-1-carboxylic acid, Ac₆c 1-aminocyclohexane-1-carboxylic acid.^{[i]}
Fig. 3 is a schematic representation of the cystine-knot motif where two disulfide bridges along with a connecting backbone form a ring, which is penetrated by the third disulfide bond; the S-S bonds are pointed with arrows, the Latin numbers of Cys residues illustrate connectivities specific for different types of cystine knots, with the Latin number before the bracket corresponding to ICK and CCK, and with the Latin number in brackets corresponding to GFCK. Illustration is modified from http://knottin.cbs.cnrs.fr.
Fig. 4 shows a model of the open chain MCoTI-II analog oMcoTI-II, deduced from the NMR structure of cyclic McoTI; the P1 position, the *N*- and C-termini and the disulfide bonds C I-C IV, C II-C V, C III-C VI are indicated. β-Strands are drawn as arrows.
Fig. 5 shows the HPLC profile of the crude peptide oMcoTIAibHar
Fig. 6 shows the results of ESI-MS analysis of the linear precursor NC-MC-PTH-1 *.
Fig. 7 shows the folding as monitored by HPLC at 215 nm.
Fig. 8 shows the results of ESI-MS analysis of the folded oMcoTIAibHa
Fig. 9 shows the HPLC profile of the crude peptide NC-MC-PTH-2.
Fig. 10 depicts the results of HPLC analysis of oxidized peptide NC-MC-PTH-2.
Fig. 11 depicts the results of high Resolution ESI-MS analysis of the final product NC-MC-PTH-2.

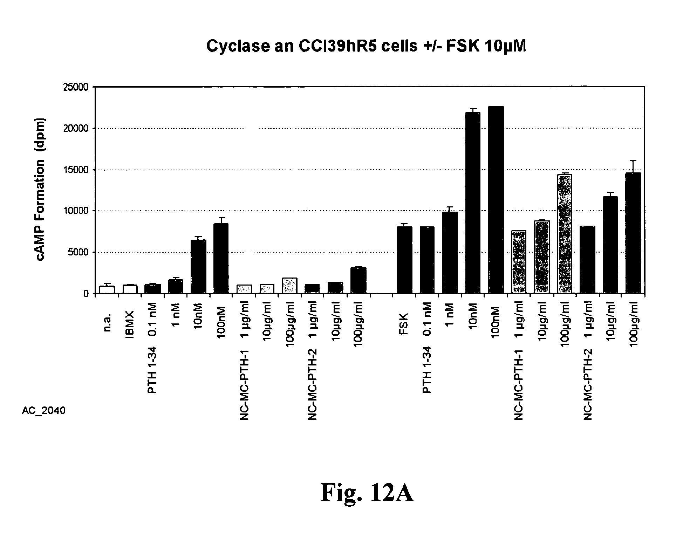

Fig. 12A depicts the results of functional assays using the PTH-receptor-expressing CC139hR5 cells to determine the degree of stimulation of the PTH-receptor in response to PTH(1-34) and NC-MC-PTH-1* and -2 as judged by the amount of cAMP produced in the absence and in the presence of Forskolin.

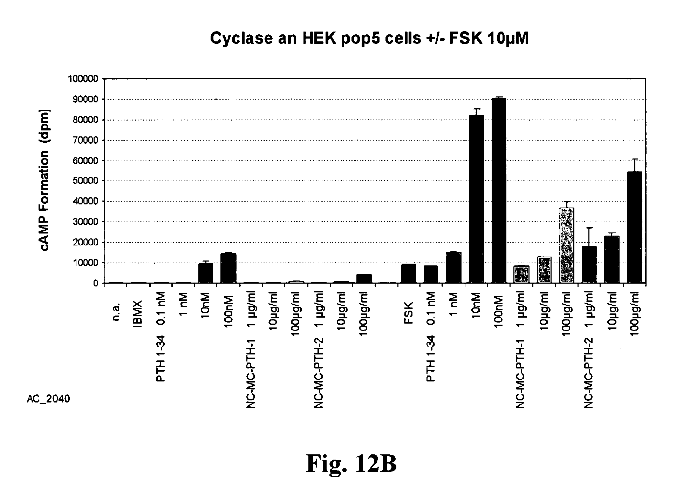

Fig. 12B depicts the results of functional assays using the PTH-receptor-expressing HEK293 cells to determine the degree of stimulation of the PTH-receptor in response to PTH(1-34) and NC-MC-PTH-1 * and -2 as judged by the amount of cAMP produced in the absence and in the presence of Forskolin.

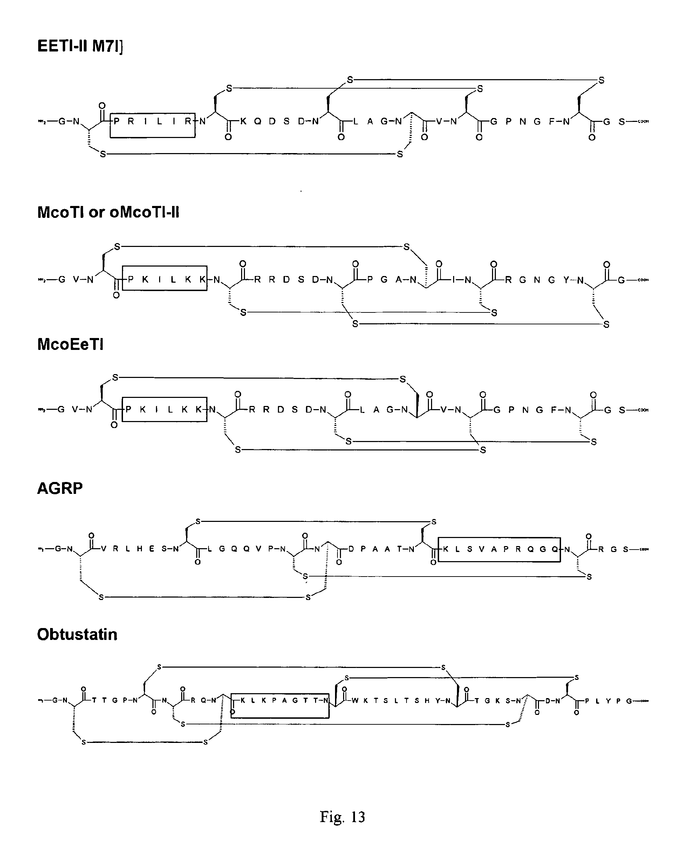

Fig. 13 represents the primary amino acid sequence including the disulfide bridges of various knottin proteins which may provide the scaffold of the polypeptide of the present invention, whereby those amino acids of the knottin protein which are deleted therefrom so as to provide a scaffold moiety as used in connection with the present invention, are boxed.

### EXAMPLE 1: Chemical synthesis of NC-MC-PTH-1 * and -2:

### Materials and Methods

All the chemicals used were of the highest grade available. Solvents were of analytical grade and used as supplied. N_{α}-Fmoc protected amino acids were used with the following side-chain protecting groups: t-Bu (Asp, Tyr), Boc (Lys), Trt (Cys, Asn), Pbf (Arg). Pseudo-proline dipeptide Fmoc-Asp(O*^{t}*Bu)-Ser(ψ^{Me,Me})pro-OH was purchased from Calbiochem-Novabiochem GmbH. ESI mass spectra were measured with a TSQ 700 Finnegan spectrometer. High-resolution ESI mass spectra were recorded with a Bruker APEX-Q III 7T. HPLC were performed on a Pharmacia Äkta basic system using YMC J'sphere ODS H-80, RP C-18 columns for preparative runs (250 x 4.6 mm, 4 µm, 80 Å) and for the analytical samples (250 x 4.6 µm, 80 Å).

### SPPS of NC-MC-PTH- 1 * and -2

In spite of visible progress in Boc-SPPS of microproteins, this approach has several disadvantages. Yields are usually quite low and the single step monitoring during automated synthesis is hampered due to the lack of a suitable chromophore. For an efficient generation of these microproteins a stepwise control of the synthesis progression is advantageous, especially when non-natural amino acids are incorporated. Moreover, Fmoc-based synthesis eliminates strong acidic cleavage, overall providing milder synthesis conditions. Therefore, the microproteins were produced by SPPS based on the Fmoc-protection strategy being aware of possible aggregation phenomena with longer peptides.

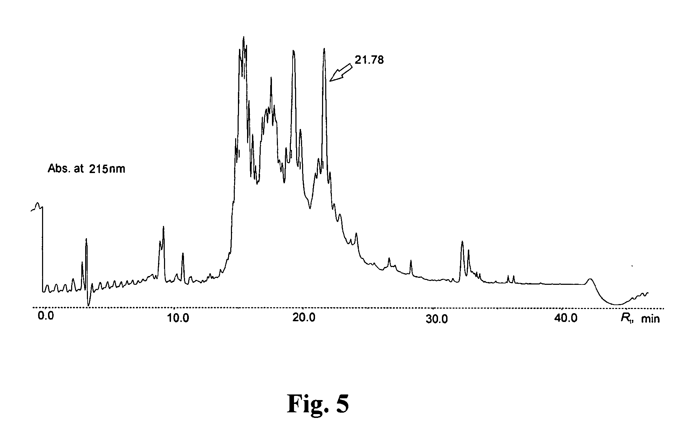

The synthesis of the microproteins was divided into two parts. The first 20 amino acids were assembled by standard automated synthesis using the peptide synthesizer *ABI* 433 A applying a special cycle for the safe Cys incorporation. This part of the proteins up to cysteine II of oMcoTI-II was called *Zagotovka* ( 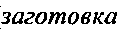 is the Russian word meaning rough stock, semifinished product). The resin was divided into two parts and the next amino acids were assembled with a manual protocol. We started with the shortest sequence PTH-1. After the automated synthesis of *Zagotovka* was completed and *N*-terminal Fmoc group was cleaved, the Fmoc-Har-OH building block was coupled manually using HATU/DIEA (*O*-(7-azabenzotriazol-1-yl)-1,1,3,3-tetramethyluroniumhexafluorophosphate/ ethyl diisopropyl amine or Hünig base) *via in situ* activation in NMP (*N*-methylpyrrolidinone ) within 1.5 h at ambient temperature. *Kaiser* test with ninhydrin showed incomplete coupling but no double coupling with fresh reagents was conducted because this commercially available building block was too expensive. After capping with Ac₂O and Fmoc deprotection the chain was assembled automatically up to Aib residue using HCTU/DIEA activation in dimethylformamide (DMF), with double couplings for two glutamines. The activator (3.9 eq. excess according to the calculating program of the *ABI* 433 Peptide synthesizer) was added as solid directly into the amino acid cartridge, and the activating mixture containing usually HBTU/HOBt (*O-*(*1H-*benzotriazol-1-yl)-1,1,3,3-tetramethyluroniumhexafluorophosphate / 1-hydroxybenzotriazol) solution was replaced with pure DMF. Delivery times were programmed based on the results of the *Flow test D* conducted with pure DMF. Aib residues were coupled manually using the same conditions as for the Har building block. The *N-*terminal part GVCP was coupled automatically using an orthogonal program for the coupling of Cys. After the assembly of the peptide chain was completed, the peptide was dried and cleaved with TFA-scavengers mixture. The HPLC analysis of the crude peptide showed rather low quality of the product; nevertheless, the mixture was quite separable and yielded after purification the target product in a marked peak (Fig. 5).

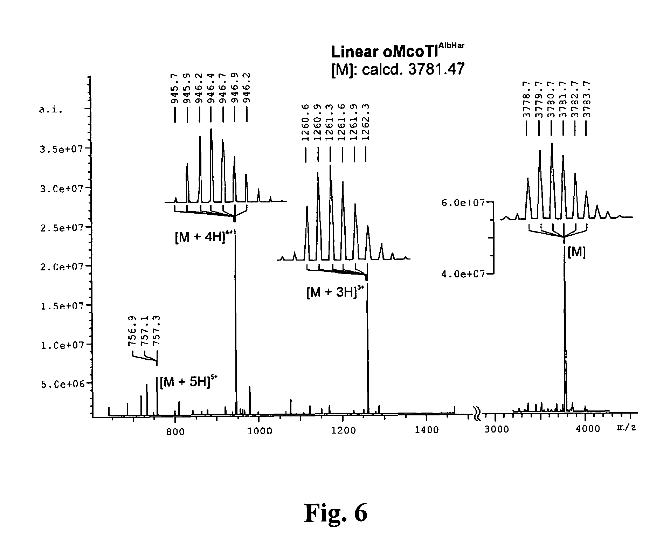

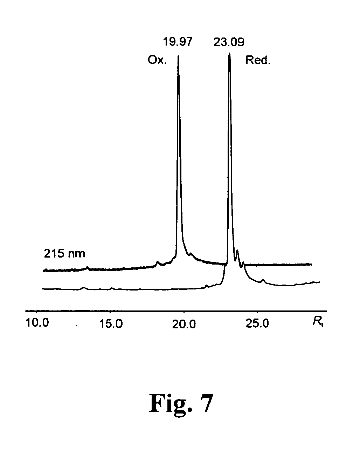

After the linear precursor was purified and analyzed with ESI-MS that showed the presence of four- and three-fold charged fragments as well as the mass peak (Fig. 6), the oxidation was conducted as described later on (Folding procedure) to give the folded product in surprisingly high quality and yield (Fig. 7).

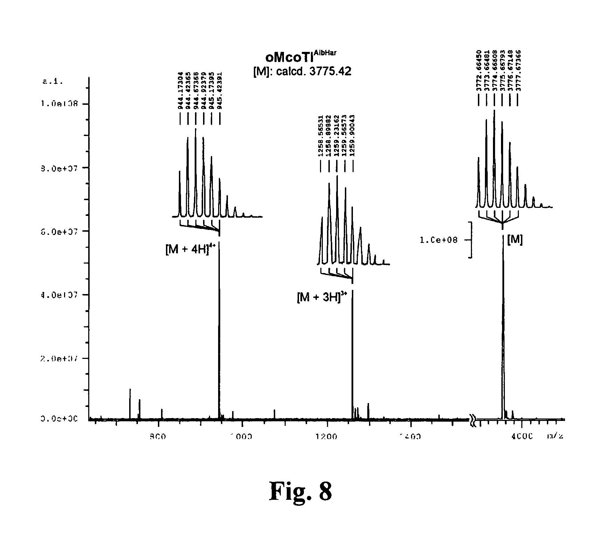

The oxidized product showed the behaviour typical of the members of the knottin family: it was eluted earlier as the reduced linear precursor. The ESI-MS (Fig. 8) showed the oxidized peptide (6 Da less as the linear one), and no signal was detected in the area where the possible dimer (containing two intramolecular disulfide bridges in each monomer, and one intermolecular bond) would be expected. This confirms that the insertion of the helix does not compromise the structure or affect the properties of the original knottin scaffold.

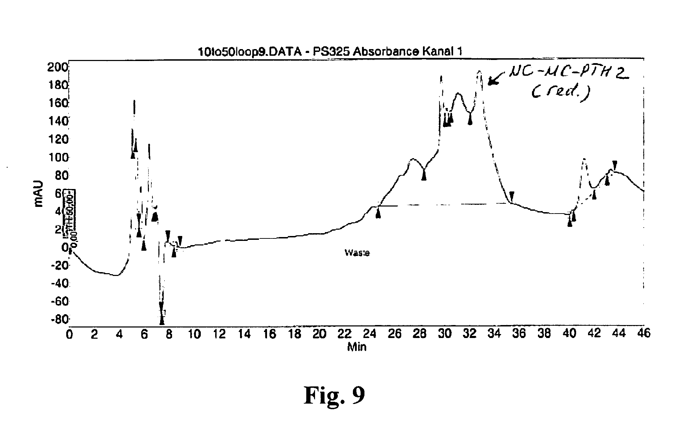

Synthesis of peptide NC-MC-PTH-1 * starting from *Zagotovka* was much more difficult than the first synthesis. Different techniques of peptide synthesis were applied to this difficult sequence to introduce the Sequence **AibVAibEIQLMHQHarAKY**. The attempt to synthesize this polypeptide by standard Fmoc synthesis, either by automated or manual synthesis was unsuccessful. The two Aib residues were expected to be the reason for this failure. In a second approach, Fmoc-Har-OH building block of the PTH sequence was coupled twice using HATU/DIEA *via in situ* activation in NMP within 1.5 h at ambient temperature. After capping with Ac2O and Fmoc deprotection the chain was assembled manually up to Aib residue using HCTU/DIEA activation in DMF, with double couplings for all amino acids. Aib residues were double coupled using HATU/DIEA activation in microwave reactor for manual peptide synthesis at 20 W and 50°C within 10 min per building block. The *N*-terminal part GVCP was assembled using microwave-assisted SPPS at 20 W and 40°C with 5 min per amino acid single coupling, except for Fmoc-Pro-OH that was coupled twice. After the assembly of the peptide chain was completed, the peptide was dried and cleaved with TFA scavenger mixture in microwave reactor at 20 W and 38°C within 20 min. The product was purified by HPLC from the crude mixture after synthesis (Fig. 9) to yield 4 mg of NC-MC-PTH2.

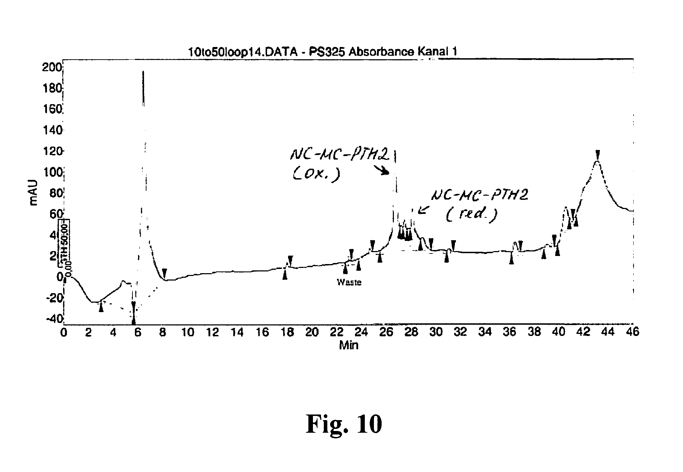

All the peaks collected during the preparative HPLC were dissolved in folding buffer and after overnight incubation analysed by HPLC (Fig. 10).
Only one peak showed the behaviour typical for oxidation of the members of the knottin family and was eluted earlier than the reduced linear precursor. The yield of the oxidized product is 1.6 mg.

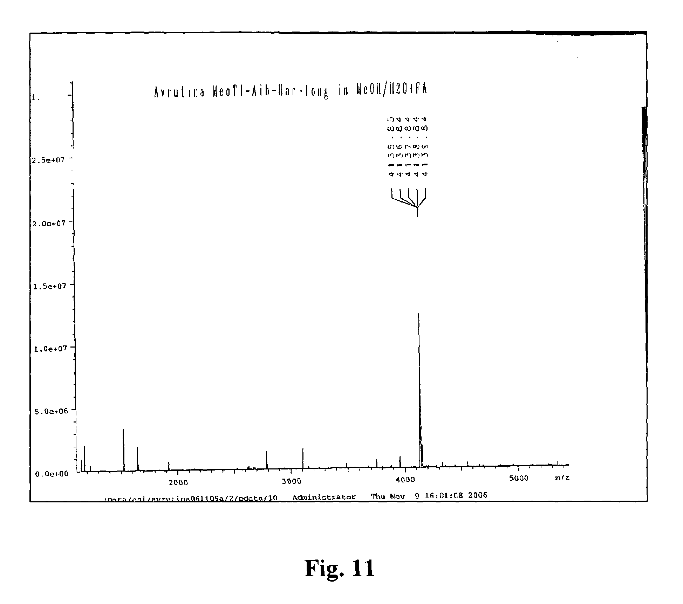

The final oxidized and purified NC-MC-PTH-1 * was subjected to high resolution ESI-MS (Fig. 11). The calculated Molecular weight is 4138,84 (oxidized form), the experimentally determined Molecular weight is 4138,84.

### Folding procedure

Oxidation of the linear PTH microbodies to the cystin-knot was performed by dissolving the reduced lyophilized peptide in 50 µl 10 mM HCl per mg of peptide followed by addition of NH₄HCO₃ (200 mM, pH 9.1) to a final concentration of 1-1.5 mg/ml (Wentzel, A., Christmann, A., Krätzner, R. and Kolmar, H. (1999): Sequence requirements of the GPNG b-turn of the Ecballium elaterium trypsin inhibitor II explored by combinatorial library screening. J. Biol. Chem. 274, 21037-21043). The reaction mixture was incubated overnight in a PET container under vigorous shaking at room temperature. Purification of folded peptide was done by RP-HPLC using Phenomenex C₁₈ columns (analytical: 250 x 4.60 mm; preparative: 250 x 10.00 mm). Conditions were as follows: Eluent A: H₂O containing 0.1% TFA, eluent B: 50 % acetonitrile, 50 % 2-propanole containing 0.1 % (v/v) TFA. A linear gradient of 10-37 % B was performed with flow rates of 1 ml/min for analytical purposes and 3.5 ml/min for preparative runs, respectively. With monitoring at 217 nm the oxidized peptide containing fraction was collected and lyophilized. Successful oxidation was confirmed by ESI mass spectrometry.

### EXAMPLE 2: Activation of type 1 Parathyroid Hormone Receptor (PTHR1) by PTH fragments inserted into knottin scaffolds

### Materials and Methods

Activation of the type 1 Parathyroid Hormone Receptor (PTHR1) was assessed using Human Embryonic Kidney 293 cells (HEK293) and hamster lung fibroblasts (CCL39) expressing the recombinant human receptor. Confluent cell cultures grown in 24 well plates were labelled with [³H]adenine (100 MBq/ml; Amersham, Zuerich, Switzerland) for 4h in serum-free DMEM medium. Cells were then incubated at 37°C in buffered salt solution containing 130 mM NaCl, 0.9 mM NaH₂PO₄, 5.4 mM KCI, 0.8 mM MgSO₄, 1.8 mM CaCl₂, 25 mM glucose. The phosphodiesterase inhibitor isobutylmethylxanthine (IBMX, 1 mM) was added to allow accumulation of cAMP. Where indicated, Forskolin (FSK, 10 µM) was added to stimulate adenylyl cyclase in synergy with peptide agonists. Incubation time was 15 minutes. Cells were then extracted with ice-cold trichloroacetic acid and cAMP separated from free adenine and ATP using batch column chromatography according to the method described by Salomon, Y. (1979): Adenylate cyclase assay. Adv. Cyclic Nucleotide Res. 10, 35-55. Methods for the quantification of cAMP are, among others, described in Allen, Mm, Hall, D., Collins., B., Moore., K. (2002) J. Biomol. Screen. 7, 35-44; Golla, R., Seethala, R.. J. Biomol. Screen. 7, 515-25; Gabriel, D., Vernier, M., Pfeifer, M.J., Dasen, B., Tenaillon, L., Bouhelal, R. ( 2003) 1, 291-303; Kent, T.C., Thompson, K.S., Naylor, L.H.. (2005) J Biomol Screen. 5, 437-46.

### Activation of PTHR1 by PTH1 * and PTH2 inserted into a Knottin scaffold (oMcoTi-II)

In order to confirm that PTH N-terminal fragments inserted into select knottin scaffolds retained their biological activity, the ability of two constructs NC-MC-PTH-1 * and -2 to elicit the biological response normally provided by PTH(1-34), the biologically active N-terminal fragment of PTH approved for clinical use), was assessed. Briefly, two mammalian cell lines that functionally express the human Parathyroid Hormone receptor (hPTH), CC139hR5 (hamster fibroblasts) and Human Embryonic Kidney cells (HEK293), were treated with various concentrations of both constructs as well as PTH (1-34) for a positive control.

When the effect of both constructs on CC139hR5 cells was tested, a significant stimulation of the cAMP production was found if high concentrations (100 µg/ml) of either NC-MC-PTH-1* and -2 were added, both in the presence and in the absence of Forskolin. The effect was even more pronounced when HEK293 cells were used, in particular when 100 µg/ml of NC-MC-PTH2 were applied which resulted in the production of approximately 60% of cAMP produced at the highest concentration of PTH 1-34 (100 nM). Overall, it is clear that high concentrations of NC-MC-PTH-1 * and -2 are able to stimulate the adenylate cyclase activated by PTH 1-34.

The features of the present invention disclosed in the specification, the claims and/or the drawings may both separately and in any combination thereof be material for realizing the invention in various forms thereof.

## Claims

1. A polypeptide comprising a scaffold moiety and a helix moiety, whereby
the helix moiety is inserted into the scaffold moiety,
the scaffold moiety comprises a knottin protein or at least one fragment thereof,
and the amino acid sequence of the polypeptide differs from the amino acid sequence of the knottin protein or at least one fragment thereof.

2. A polypeptide comprising a scaffold moiety and a biologically active peptide moiety, whereby
the biologically active peptide moiety is inserted into the scaffold,
the scaffold moiety comprises a knottin protein or at least one fragment thereof,
and the amino acid sequence of the polypeptide differs from the amino acid sequence of the knottin protein or at least one fragment thereof.

3. The polypeptide according to any of claims 1 or 2, wherein the polypeptide and/or the scaffold moiety is cyclic.

4. The polypeptide according to any of claims 1 or 2, wherein the polypeptide and/or the scaffold moiety is linear.

5. The polypeptide according to any of claims 1 to 4, wherein the knottin protein is selected from the group comprising EETI-II M7I, oMcoTI-II, McoEeTI, AGRP' and Obtustatin.

6. The polypeptide according to any of claims 1 to 5, wherein the helix moiety comprises an amino acid sequence, whereby such amino acid sequence is one of a biologically active peptide, whereby preferably such biologically active peptide is selected from the group comprising peptide hormones, cytokines, integrins, integrin ligands protease inhibitors, GPCR ligands, ion chanel ligands, DNA or RNA ligands, viral proteins, bacterial proteins or a fragment and/or derivative thereof.

7. The polypeptide according to any of claims 1 to 5, wherein the biologically active peptide moiety comprises an amino acid sequence whereby such amino acid sequence is one of a peptide selected from the group comprising peptide hormones, cytokines, integrins, protease inhibitors, viral proteins, bacterial proteins or a fragment and/or derivative thereof.

8. The polypeptide according to any of claims 1 to 7, wherein the amino acid sequence of the scaffold moiety comprises at least two, preferably at least four and more preferably six Cys residues.

9. The polypeptide according to any of claims 1 to 7, wherein the amino acid sequence of the scaffold moiety comprises six or eight cysteines.

10. The polypeptide according to any of claims 1 to 9, wherein the helix moiety or the biologically active peptide moiety is inserted into the scaffold moiety between two Cys redidues of the knottin protein of the scaffold moiety.

11. The polypeptide according to any of claims 1 to 10, wherein the helix moiety or the biologically active peptide moiety is inserted into the scaffold moiety, counting from the N-terminus to the C-terminus, between the first and the second, the fourth and the fifth or the fifth and the sixth Cys residue of the scaffold moiety.

12. The polypeptide according to any of claims 8 to 11, wherein the scaffold moiety is derived from the knottin protein by having deleted at least one, preferably more and most preferably all of the amino acid residues between the Cys residues of the knottin protein or a fragment thereof between which the helix moiety or the biologically active peptide moiety is inserted.

13. The polypeptide according to claim 12, wherein all of the amino acid residues between the Cys residues of the knottin protein or the fragment thereof are deleted.

14. The polypeptide according to any of claims 1 to 13,
wherein the knottin protein is EETI-II M7I and the amino acid sequence between the first and the second cysteine of the knottin protein is partially or in its entirety replaced by the amino acid sequence of the helix moiety or the biologically active peptide moiety, or
wherein the knottin protein is oMcoTI-II and the amino acid sequence between the first and the second cysteine of the knottin protein is partially or in its entirety replaced by the amino acid sequence of the helix moiety or the biologically active peptide moiety, or
wherein the knottin protein is McoEeTI and the amino acid sequence between the first and the second cysteine of the knottin protein is partially or in its entirety replaced by the amino acid sequence of the helix moiety or the biologically active peptide moiety, or
wherein the knottin protein is AGRP' and the amino acid sequence between the fifth and the sixth cysteine of the knottin protein is partially or in its entirety replaced by the amino acid sequence of the helix moiety or the biologically active peptide moiety, or
wherein the knottin protein is Obtustatin and the amino acid sequence between the fourth and the fifth cysteine of the knottin protein is partially or in its entirety replaced by the amino acid sequence of the helix moiety or the biologically active peptide moiety.

15. The polypeptide according to any of claims 1 to 9, wherein the helix moiety or the biologically active peptide moiety is fused to the N-terminus or the C-terminus of the scaffold moiety.

16. The polypeptide according to any of claims 1 to 15, wherein the biologically active peptide is selected from the group comprising PTH, PTH derivatives and PTH analogues, preferably selected from the group comprising a peptide having an amino acid sequence according to SEQ. ID No. 1, a peptide having an amino acid sequence according to SEQ. ID No. 2 and a peptide having an amino acid sequence according to SEQ. ID. No. 3, and a peptide having an amino acid sequence according to SEQ. ID. No. 12 and fragments and/or derivatives thereof.

17. The polypeptide according to any of claims 1 to 15, wherein the scaffold moiety is oMcoTI-II according to SEQ ID No 4 and the helix moiety or the biologically active peptide moiety is a peptide having an amino acid sequence according to SEQ ID No. 2 or an amino acid according to SEQ. ID. No. 12, with the helix moiety of the biologically active peptide moiety being inserted between the first cysteine and the second cysteine of the amino acid sequence according to SEQ ID No 4, replacing the amino acid residues occurring between said two cysteines of the amino acid sequence of the knottin protein.

18. The polypeptide according to any of claims 1 to 15, wherein the scaffold moiety is oMcoTI-II according to SEQ ID No 4 and the helix moiety or the biologically active peptide moiety is a peptide having an amino acid sequence according to SEQ ID No. 3, with the helix moiety of the biologically active peptide moiety being inserted between the first cysteine and the second cysteine of the amino acid sequence according to SEQ ID No 4, replacing the amino acid residues naturally occurring between said two cysteines of the amino acid sequence of the knottin protein.

19. The polypeptide according to any of claims 1 to 18, wherein the length of the helix moiety and /or the biologically active peptide moiety is from about 4 to 30 amino acids, preferably from about 4 to 25 amino acids, more preferably from about 4 to 20 amino acids, and even more preferably from about 4 to 15 amino acids.

20. The polypeptide according to any of the preceding claims, whereby the polypeptide comprises an amino acid sequence according to SEQ. ID. No. 6, SEQ. ID. No. 7 or SEQ. ID. No. 13.

21. The polypeptide according to any of claims 1 to 20, wherein the polypeptide is a recombinant protein.

22. The polypeptide according to any of claims 1 to 20, wherein the polypeptide is a chemically synthesised protein or a synthetic protein.

23. The polypeptide according to any of claims 1 to 22, wherein the scaffold moiety comprises one knottin protein or at least one fragment thereof.

24. The polypeptide according to any of claims 1 to 22, wherein the scaffold protein comprises a multimer of a knottin protein or at least a fragment thereof, preferably a dimer.

25. The polypeptide according to claim 23 or 24, whereby the knottin protein or at least one fragment thereof is a knottin protein or at least one fragment thereof as defined in any of claims 1 to 21.

26. A pharmaceutical composition comprising a polypeptide according to any of claims 1-25 and a pharmaceutically acceptable carrier.

27. The pharmaceutical composition according to claim 26, wherein the pharmaceutical composition is for oral administration.

28. Use of the polypeptide according to any of claims 1 to 25 for the manufacture of a medicament for the treatment or the prevention of a disease.

29. Use of the polypeptide according to any of claims 1 to 25 for the manufacture of a diagnostic agent for the diagnosis of a disease.

30. Use according to any of claims 28 or 29, wherein the disease is bone-related disorder.

31. Use according to claim 30, wherein the bone-related disorder is a bone disorder **characterized by** low bone mineral density (BMD) and/or bone fragility.

32. Use according to claim 31, wherein the bone disease is selected from the group comprising primary and secondary osteoporosis, osteopenia, osteomalacia, osteogenesis imperfecta (OI), avascular necrosis (osteonecrosis), fractures and implant healing, bone loss due to other disorders.

33. Use according to claim 32, wherein the other disorder resulting in bone loss is selected from the group comprising HIV infection, cancers and arthritis.

34. Use according to claim 32, wherein the implant healing is the implant healing of dental implants or hip implants.

35. Use according to claim 31, wherein the bone-related disorder is selected from the group comprising osteoarthritis, arthritis, and the formation and presence of osteolytic lesions.


SEQUENCE LISTING

<110> NascaCell Technologies AG

<120> Polypeptide comprising a knottin protein moeity

<130> N 19022 EP

<160> 13

<170> PatentIn version 3.1

<210> 1
<211> 84
<212> PRT
<213> Homo sapiens

<400> 1

Ser Val Ser Glu Ile Gln Leu Met His Asn Leu Gly Lys His Leu Asn
1               5                   10                  15

Ser Met Glu Arg Val Glu Trp Leu Arg Lys Lys Leu Gln Asp Val His
            20                  25                  30

Asn Phe Val Ala Leu Gly Ala Pro Leu Ala Pro Arg Asp Ala Gly Ser
        35                  40                  45

Gln Arg Pro Arg Lys Lys Glu Asp Asn Val Leu Val Glu Ser His Glu
    50                  55                  60

Lys Ser Leu Gly Glu Ala Asp Lys Ala Asp Val Asn Val Leu Thr Lys
65                  70                  75                  80

Ala Lys Ser Gln

<210> 2
<211> 14

<212>  PRT

<213>  Artificial Sequence

<220>

<223>  synthetic PTH1

<220>

<221>  artificial amino acid

<222>  (1)..(1)

<223>  alpha-Aminoisobutyric acid

<220>

<221>  artificial amino acid

<222>  (3)..(3)

<223>  alpha-Aminoisobutyric acid

<220>

<221>  artificial amino acid

<222>  (11)..(11)

<223>  homoarginine

<400>  2

Ala Val Ala Glu Ile Gln Leu Met His Gln Arg Ala Lys Trp
1               5                   10

<210>  3

<211>  11

<212>  PRT

<213>  Artificial Sequence

<220>

<223>  synthetic PTH2

<220>

<221>  artificial amino acid

<222>  (1)..(1)

<223>  alpha-Aminoisobutyric acid


<220>

<221>  artificial amino acid

<222>  (3)..(3)

<223>  alpha-Aminoisobutyric acid


<220>

<221>  artificial amino acid

<222>  (11)..(11)

<223>  homoarginine


<400>  3

Ala Val Ala Glu Ile Gln Leu Met His Gln Arg
1               5                   10


<210>  4

<211>  29

<212>  PRT

<213>  Momordica cochinchinensis


<400>  4

Gly Val Cys Pro Lys Ile Leu Lys Lys Cys Arg Arg Asp Ser Asp Cys
1               5                   10                  15

Pro Gly Ala Cys Ile Cys Arg Gly Asn Gly Tyr Cys Gly
            20                  25


<210>  5

<211>  37

<212>  PRT

<213>  artificial sequence

<220>

<223>  synthetic NC-MC-PTH1

<220>

<221>  artificial amino acid

<222>  (4)..(4)

<223>  alpha-Aminoisobutyric acid

<220>

<221>  artificial amino acid

<222>  (6)..(6)

<223>  alpha-Aminoisobutyric acid

<220>

<221>  artificial amino acid

<222>  (14)..(14)

<223>  homoarginine

<400>  5

Gly Val Cys Ala Val Ala Glu Ile Gln Leu Met His Gln Arg Ala Lys
1               5                   10                  15

Trp Cys Arg Arg Asp Ser Asp Cys Pro Gly Ala Cys Ile Cys Arg Gly
            20                  25                  30

Asn Gly Tyr Cys Gly
        35

<210>  6

<211>  37

<212>  PRT

<213>  artificial sequence

<220>

<223>  synthetic NC-MC PTH1

<220>

<221>  artificial amino acid

<222>  (4)..(4)

<223>  alpha-Aminoisobutyric acid


<220>

<221>  artificial amino acid

<222>  (6)..(6)

<223>  alpha-Aminoisobutyric acid


<220>

<221>  artificial amino acid

<222>  (14)..(14)

<223>  homoarginine


<400>  6

Gly Val Cys Ala Val Ala Glu Ile Gln Leu Met His Gln Arg Ala Lys
1               5                   10                  15

Trp Cys Arg Arg Asp Ser Asp Cys Pro Gly Ala Cys Ile Cys Arg Gly
            20                  25                  30

Asn Gly Tyr Cys Gly
        35

<210>  7

<211>  34

<212>  PRT

<213>  artificial sequence


<220>

<223>  synthetic NC-MC PTH2

<220>

<221>  artificial amino acid

<222>  (4)..(4)

<223>  alpha-Aminoisobutyric acid

<220>

<221>  artificial amino acid

<222>  (6)..(6)

<223>  alpha-Aminoisobutyric acid

<220>

<221>  artificial amino acid

<222>  (14)..(14)

<223>  homoarginine

<400>  7

Gly Val Cys Ala Val Ala Glu Ile Gln Leu Met His Gln Arg Cys Arg
1               5                   10                  15

Arg Asp Ser Asp Cys Pro Gly Ala Cys Ile Cys Arg Gly Asn Gly Tyr
            20                  25                  30

Cys Gly

<210>  8

<211>  29

<212>  PRT

<213>  Ecbalium elaterium

<400>  8

Gly Cys Pro Arg Ile Leu Ile Arg Cys Lys Gln Asp Ser Asp Cys Leu
1               5                   10                  15

Ala Gly Cys Val Cys Gly Pro Asn Gly Phe Cys Gly Ser
            20                  25

<210>  9

<211>  30

<212>  PRT

<213>  Momordia cochinchinensis

<400>  9

Gly Val Cys Pro Lys Ile Leu Lys Lys Cys Arg Arg Asp Ser Asp Cys
1               5                   10                  15

Leu Ala Gly Cys Val Cys Gly Pro Asn Gly Phe Cys Gly Ser
            20                  25                  30

<210>  10

<211>  37

<212>  PRT

<213>  Homo sapiens

<400>  10

Gly Cys Val Arg Leu His Glu Ser Cys Leu Gly Gln Gln Val Pro Cys
1               5                   10                  15

Cys Asp Pro Ala Ala Thr Cys Lys Leu Ser Val Ala Pro Arg Gln Gly
            20                  25                  30

Gln Cys Arg Gly Ser
        35

<210>  11

<211>  42

<212>  PRT

<213>  Vipera lebetina obtusa

<400>  11

Gly Cys Thr Thr Gly Pro Cys Cys Arg Gln Cys Lys Leu Lys Pro Ala
1               5                   10                  15

Gly Thr Thr Cys Trp Lys Thr Ser Leu Thr Ser His Tyr Cys Thr Gly
            20                  25                  30

Lys Ser Cys Asp Cys Pro Leu Tyr Pro Gly
        35                  40

<210>  12

<211>  14

<212>  PRT

<213>  artificial sequence

<220>

<223>  synthetic PTH1*

<220>

<221>  artificial amino acid

<222>  (1)..(1)

<223>  alpha-Aminoisobutyric acid

<220>

<221>  artificial amino acid

<222>  (3)..(3)

<223>  alpha-Aminoisobutyric acid

<220>

<221>  artificial amino acid

<222>  (11)..(11)

<223>  homoarginine

<400>  12

Ala Val Ala Glu Ile Gln Leu Met His Gln Arg Ala Lys Tyr
1               5                   10

<210>  13

<211>  37

<212>  PRT

<213>  artificial sequence

<220>

<223>  synthetic NC-MC PTH1*

<220>

<221>  artificial amino acid

<222>  (4)..(4)

<223>  alpha-Aminoisobutyric acid

<220>

<221>  artificial amino acid

<222>  (6)..(6)

<223>  alpha-Aminoisobutyric acid

<220>

<221>  artificial amino acid

<222>  (14)..(14)

<223>  homoarginine

<400>  13

Gly Val Cys Ala Val Ala Glu Ile Gln Leu Met His Gln Arg Ala Lys
1               5                   10                  15

Tyr Cys Arg Arg Asp Ser Asp Cys Pro Gly Ala Cys Ile Cys Arg Gly
            20                  25                  30

Asn Gly Tyr Cys Gly
        35

7

9